# EUROPEAN PATENT SPECIFICATION

(11) **EP 0 450 472 B1**
(45) Date of publication and mention of the grant of the patent: **14.05.1997**
(21) Application number: 91104816.3
(22) Date of filing: 26.03.1991
(51) Int. Cl.: C12P 21/08, A61K 39/395

(54) **Monoclonal antibodies that inhibit growth of kaposi's sarcoma**
Monoklonale Antikörper, die das Wachstum des Karposisarkomas inhibieren
Anticorps monoclonaux inhibant la croissance du sarcome de kaposi

(30) Priority: 29.03.1990 US 502141
(43) Date of publication of application: 09.10.1991
(73) Proprietor: ONCOGEN LIMITED PARTNERSHIP, Seattle, WA 98121 (US); UNITED STATES GOVERNMENT as represented by THE SECRETARY OF THE DEPARTMENT OF HEALTH AND HUMAN SERVICES, Bethesda, MD 20892 (US)
(72) Inventor: Salahuddin, Syed Zaki, South Pasadena, California 91030 (US); Nakamura, Shuji, South Pasadena, California 91030 (US); Gallo, Robert C., Bethesda, Maryland 20817 (US); Radka, Susan F., Seattle, Washington 98103 (US); Linsley, Peter S., Seattle, Washington 98119 (US); Shoyab, Mohammed, Seattle, Washington 98119 (US)
(74) Representative: Kinzebach, Werner, Dr.

(56) References cited:
- GB-A- 2 185 485

## Description

The present invention is directed to the use of anti-Oncostatin M monoclonal antibodies to inhibit the growth potentiating effect of Oncostatin M on Kaposi's Sarcoma, a disease associated with AIDS. The antibodies are characterized as being capable of binding to Oncostatin M, inhibiting Oncostatin M - Oncostatin M receptor binding and/or inhibiting Oncostatin M bioactivity. The monoclonal antibodies of the present invention may also be used to detect the presence of Oncostatin M and/or employed to modulate Oncostatin M biological activities in an in vivo or in vitro system.

GB 2 185 485 describes the cell growth regulatory factor Oncostatin M and refers to polypeptide fragments thereof showing immunological cross reactivity with intact Oncostatin M. These polypeptides are useful to induce antibodies to Oncostatin M which can be used to determine the concentration of Oncostatin M in a bodyfluid, to bind the Oncostatin M and thus modulate its activity and to purify Oncostatin M.

It is now well recognized that somatic cell hybrids are an important source of specific cellular products that cannot be obtained from short-term primary cultures. The best example of this is the system developed by Milstein and others for the production of hybrid myelomas for making a monoclonal antibody against an antigen of choice (Kohler and Milstein, 1985, Nature (London) 256:495; Galfre et al., 1977, Nature (London) 266:550). Such hybrids provide a constant supply of monoclonal antibody against specific antigens. These antibodies can be used as reagents for any procedure for which antibodies were previously used, but with the added advantage of higher levels of discrimination, lower background and a continuous available supply of the antibodies.

The production of monoclonal antibodies, in general, first involves immunization, removal of immune response cells, fusion of these cells in, for example, polyethylene glycol, with constantly dividing ("immortal") tumor cells selected for their inability to secrete an immunoglobin. The resulting cells are distinguished by growth in, for example, HAT (hypoxanthine, aminopterin, thymidine) media. Each hybridoma is the fusion product of a single antibody-forming cell and a tumor cell which has the ability of the former to secrete a single species of antibody and the immortality of the latter enabling it to proliferate continuously, and which provides a cell progeny with an unending supply of antibody with a single specificity.

The present invention involves the use of monoclonal antibodies specific for Oncostatin M to inhibit the growth potentiating effect of Oncostatin M on Kaposi's Sarcoma associated with AIDS. A technique for the production of the monoclonal antibodies of the invention using cell hybridization is described herein, but the scope of the invention is not intended to be restricted to the use of such cell hybridization techniques. Any monoclonal antibody or fragment thereof, having the characteristics of the monoclonal antibodies described herein are encompassed within the scope of the present invention.

The invention is described by way of examples in which hybridoma technology is used to generate the anti-Oncostatin M antibodies of the invention, which antibodies are grouped according to whether they form immuno- precipitates with either native Oncostatin M, denatured Oncostatin M, or both. Each group is characterized further by the ability to block Oncostatin M growth inhibitory bioactivity and/or the binding of Oncostatin M to its cell surface receptors.

The examples also demonstrate that neutralizing anti-Oncostatin M antibodies inhibit the growth of Kaposi's Sarcoma derived AIDS-KS cells which is potentiated and maintained by Oncostatin M or a factor found in conditioned media of an HTLV-II transformed T cell line.

The monoclonal antibodies used in the invention, or an antigenic determinant recognizing fragment thereof, can be administered to achieve therapeutically effective serum concentrations in vivo; such concentrations may be determined in vitro using the assay systems described herein.

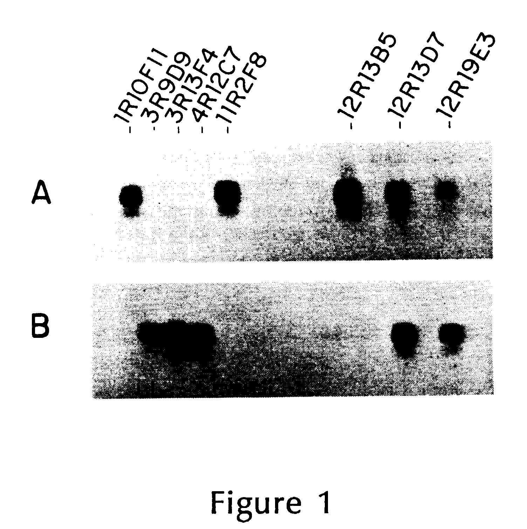

FIGURE 1 shows the immunoprecipitation of ³⁵S-methionine and ³⁵S-cysteine-labelled Oncostatin M from supernatants of the Chinese Hamster Ovary ("CHO") cell line stably transfected with cDNA encoding Oncostatin M. Figure 1A shows the reactivity of a series of anti-Oncostatin M monoclonal antibodies with "native" metabolically labelled Oncostatin M (supernatant collected from metabolically labelled CHO transfectants). Figure 1B shows the reactivity of these same antibodies with supernatant, collected from metabolically labelled CHO cells, which was denatured by treatment with SDS, 2-mercaptoethanol, and boiling prior to incubation with the monoclonal antibodies. Lane 1:negative control antibody; lane 2:1R10F11; lane 3:3R9D9; lane 4:3R13F4; lane 5:4R12C7; lane 6:11R2F8; lane 7:12R13B5; lane 8:12R13D7; lane 9:12R19E3.

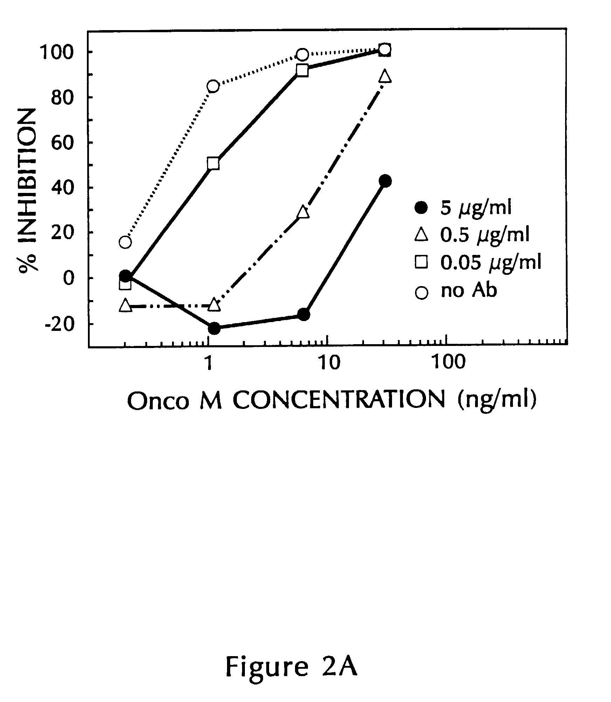

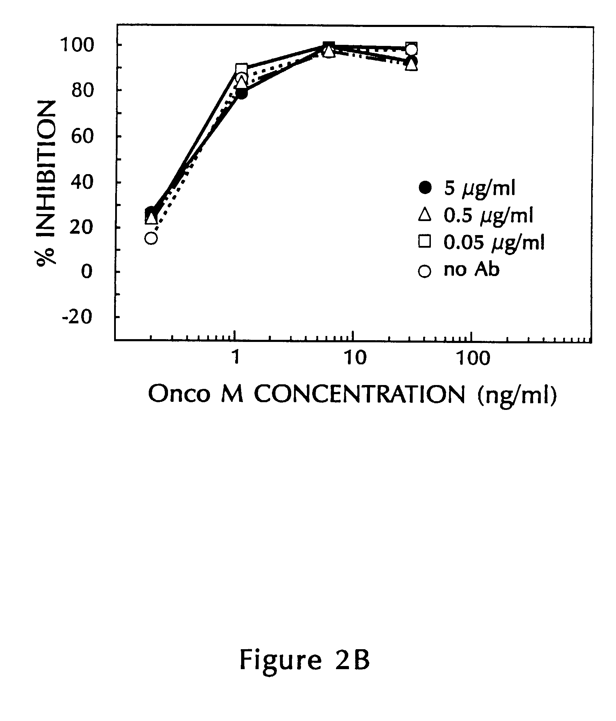

FIGURE 2 provides data from the examination of neutralizing activity of two different anti-Oncostatin M monoclonal antibodies in a Growth Inhibition Assay ("GIA") on the A375 melanoma cell line. Figure 2A shows the activity of various concentrations of 11R2F8; Figure 2B shows the activity of various concentrations of 1R10F11.

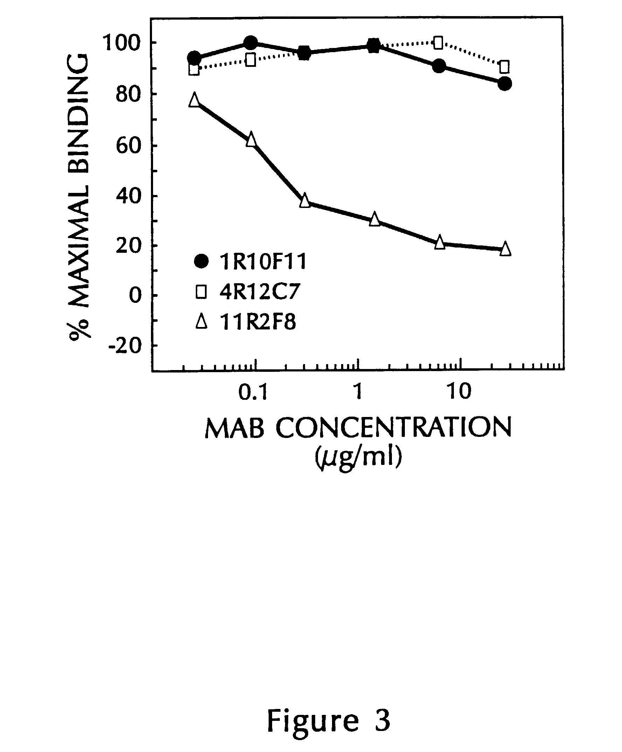

FIGURE 3 shows the effects of several anti-Oncostatin M monoclonal antibodies on the binding of ¹²⁵I-Oncostatin M to the H2981 lung carcinoma cell line in the radioreceptor assay.

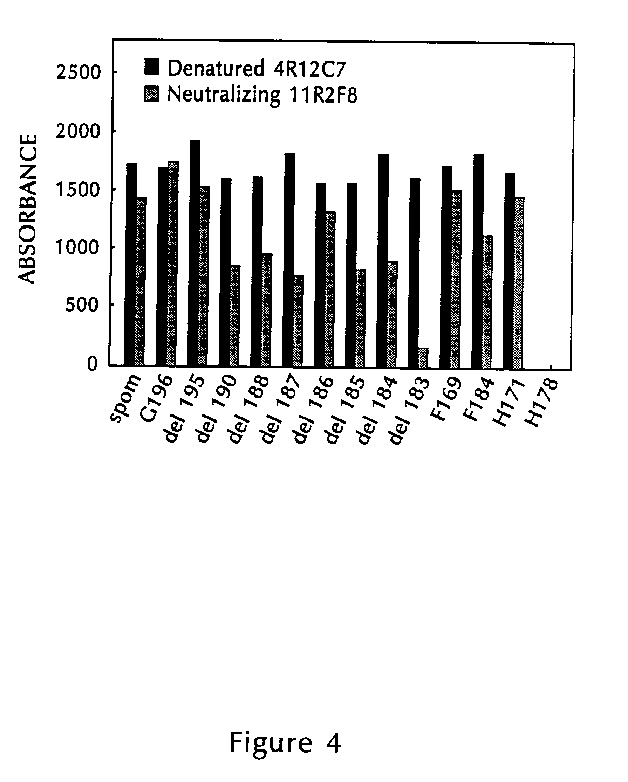

FIGURE 4 shows the binding, as detected by EIA, of two different anti-Oncostatin M monoclonal antibodies secreted by COS cells transfected with a series of mutant Oncostatin M constructs containing amino acid deletions or alterations. Data are presented as total absorbance units at OD₄₆₀. Background binding is not subtracted. In this figure, "del" indicates the last amino acid which is deleted from the C-terminus, while alphabetical letters indicate the amino acid alteration made.

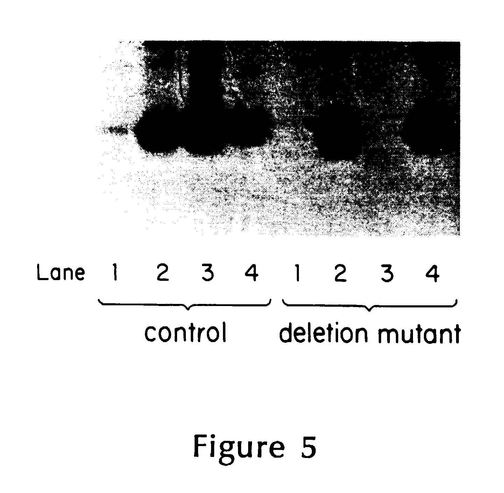

FIGURE 5 shows a comparison of the ability of different anti-Oncostatin M monoclonal antibodies to immunoprecipitate Oncostatin M secreted by either the parental construct, "SPOM", or the deletion mutant Δ44-47. Lane 1:negative control antibody; lane 2:1R10F11; lane 3:11R2F8; lane 4:12R13D7.

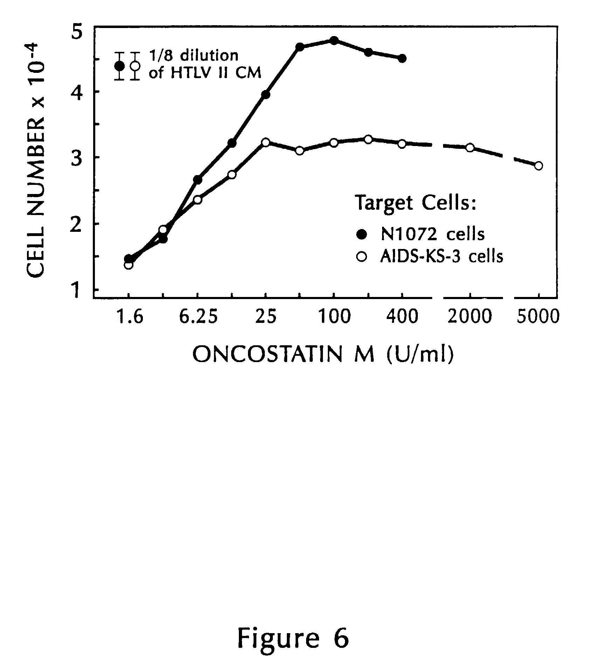

FIGURE 6 shows the growth potentiating activity of Oncostatin M on Kaposi's Sarcoma cell line (open symbols) and smooth muscle cells (N 1072 cells) derived from the pleural effusion of a patient with malignant mediastinal lymphoma (closed symbols). Recombinant Oncostatin M expressed in CHO cells was added in increasing concentrations as shown.

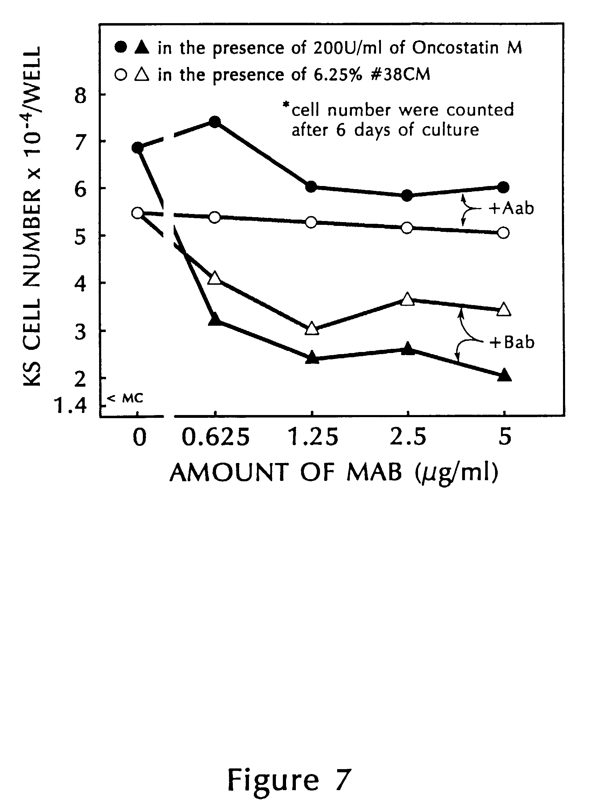

FIGURE 7 shows the effect of anti-Oncostatin M monoclonal antibodies on the growth of a Kaposi's Sarcoma cell line treated with either purified Oncostatin M (closed symbols) or conditioned medium ("CM") from #38, an HTLV-II-infected cell line (open symbols) in vitro. Either 1R10F11 (Aab) or 11R2F8 (Bab), in increasing concentrations was added to the cells cultured in the presence of purified Oncostatin M or #38 conditioned medium ("CM").

The present invention relates to the use of compositions which neutralize the activity of Oncostatin M to inhibit the growth of Kaposi's Sarcoma associated with AIDS. Monoclonal antibodies, their characterization and use are described and exemplified in the sections below.

### ONCOSTATIN M ANTIGEN RECOGNITION

Oncostatin M, originally identified for its inhibitory effects on human tumor cell lines, was first isolated from phorbol 12-myristate 13-acetate (PMA)-induced human histiocytic lymphoma cells (Zarling et al., 1986, Proc. Natl. Acad. Sci.(USA) 83: 9739-9743) and from activated T lymphocytes (Brown et al., 1987, J. Immunol. 139: 2977-2983). The molecule is a heat and acid stable protein comprised of a single polypeptide chain of Mᵣ = 28,000. Like other naturally occurring growth regulators, Oncostatin M exhibits a variety of biological activities. Growth inhibition is observed with some, but not all, human tumor cell lines. In contrast, the growth of some normal fibroblasts, such as human foreskin fibroblasts or WI-38 cells, is stimulated by exposure to Oncostatin M (Zarling et al., 1986, Proc. Natl. Acad. Sci. (USA) 83: 9739-9743). The gene for Oncostatin M has been cloned and sequenced, and an active form of recombinant Oncostatin M has recently been expressed in mammalian cells, EPA 0 290 948 (Published Nov. 17, 1988). The mature form, after cleavage of the signal peptide, is a glycoprotein containing 227 amino acids, five of which are cysteine residues. The protein has an extremely hydrophilic carboxy terminal domain. Although Oncostatin M is not structurally related to other known cytokines, its mRNA contains an AU-rich region at its 3' untranslated end. This region in the Oncostatin M message is homologous to that of many cytokines, lymphokines and other growth-regulatory molecules, suggesting a common mode of regulating gene expression. A cellular receptor for Oncostatin M has been found on a variety of mammalian cells. The major Oncostatin M receptor molecule is a specific protein of Mᵣ = 150,000-160,000 (Linsley et al., 1989, J. Biol. Chem. 264: 6528-6532).

Oncostatin M may be obtained by techniques well known in the art from a variety of cell sources which synthesize bioactive Oncostatin M including, for example, cells which naturally produce Oncostatin M and cells transfected with recombinant DNA molecules capable of directing the synthesis and/or secretion of Oncostatin M. Alternatively, Oncostatin M may be synthesized by chemical synthetic methods including but not limited to solid phase peptide synthesis. Methods for the production of Oncostatin M are described in European Patent Application, EPA 0 290 948 (published Nov. 17, 1988).

Monoclonal antibodies with an affinity for Oncostatin M may be selected by assaying their capacity for binding Oncostatin M using any of a number of immunological assays, including but not limited to, enzyme linked immunosorbant assay (ELISA), immunoprecipitation, Western blot analysis, radio-immunometric assays, competitive and non-competitive immunoassays. For example, the solid phase micro-enzyme assay (MicroEIA), described below, may be readily used. Briefly, antibodies found in the supernatant of hybrids are assessed by their ability to bind to Oncostatin M coated to a solid surface in wells. Following the addition of the supernatant, peroxidase-conjugated F(ab')₂ goat anti-mouse Ig is added to the well. After washing away any unbound material, the bound enzyme is revealed by addition of a substrate which undergoes a color change. The color change indirectly indicates a monoclonal antibody/Oncostatin M complex formed in the well.

### INHIBITION OF ONCOSTATIN M ACTIVITY

Antibodies which inhibit the biological activity of Oncostatin M may find particular use in therapeutic applications. Such antibodies can be identified using the Growth Inhibition Assay ("GIA") as described below. Briefly, GIA provides a test system to assess the ability of an antibody to neutralize the inhibitory effects of Oncostatin M on the growth and proliferation of target cells.

### INHIBITION OF BINDING TO ONCOSTATIN M RECEPTOR

Cell surface receptors generally have a high affinity for their ligand. The binding of the ligand to the specific cell surface receptor initiates the control of various cellular events. Binding of Oncostatin M to a membrane receptor has been demonstrated using the radioreceptor assay below, and in EP-A-0 290 948. The human tumor cells tested included A375 (melanoma); A875 (melanoma); Me1109 (melanoma); T24 (bladder carcinoma); A549 (lung adenocarcinoma); H1477 (melanoma); Me180 (melanoma); and MCF (breast). Binding of ¹²⁵I-Oncostatin M was specific and saturable, and was not inhibited by other known polypeptide growth regulators. Scatchard analysis of binding data obtained with different cell lines revealed that ¹²⁵I-Oncostatin M bound to 1-2x10⁴ binding sites per cell with a K_{d} of approximately 10⁻⁹ M. The monoclonal antibodies produced by the hybrid cell lines were tested, using the radioreceptor assay as described below, for their ability to block the binding of Oncostatin M to its cell surface receptor.

### ANTI-ONCOSTATIN M MONOCLONAL ANTIBODIES INHIBIT GROWTH POTENTIATING EFFECT OF ONCOSTATIN M ON KAPOSI'S SARCOMA DERIVED AIDS KS CELLS

A form of Kaposi's Sarcoma is associated with patients infected by human immunodeficiency virus type 1 (HIV-1). Although there is a clear correlation between HIV-1 infection and AIDS related Kaposi's Sarcoma, no genomic sequences of HIV-1 or of any other virus have been detected in Kaposi's Sarcoma tissue (Civantos et al., 1982, J. Invest. Dermatol. 79:79). Recently, AIDS-Kaposi's Sarcoma cells cultured in the presence of conditioned medium from an HTLV-II transformed T cell line have revealed a growth factor present in the medium which stimulated and maintained the growth of the Kaposi's Sarcoma cell line (Nakamura, S., et al. Science, 242, 426, October 21, 1988).

Experiments described below demonstrate that Oncostatin M also potentiates the growth of Kaposi's Sarcoma cells (Figure 6).

As demonstrated below, neutralizing monoclonal antibodies used in the invention (e.g., as determined by GIA analysis) inhibit the growth potentiating activity of both Oncostatin M, and the conditioned medium of the HTLV-II transformed T cell line on AIDS-KS cells (Figure 7).

### METHODS FOR PREPARING MONOCLONAL ANTIBODIES TO ONCOSTATIN M

The anti-Oncostatin M antibodies can be prepared using any of a variety of techniques in which Oncostatin M is used as an immunogen injected into a mammalian host, e.g. mouse, cow, goat, sheep, rabbit, etc., particularly with an adjuvant, e.g. complete Freund's adjuvant, aluminum hydroxide gel, or the like. The host may then be bled and the blood employed for isolation of polyclonal antibodies. Alternatively, the peripheral blood lymphocytes, splenic lymphocytes (B-cells), or lymph node lymphocytes may be employed for fusion with an appropriate myeloma cell to immortalize the chromosomes for monoclonal expression of antibodies specific for Oncostatin M.

While the invention is described by way of examples using mouse monoclonal antibodies, the invention is not so limited and encompasses the use of, for example, human antibodies. Such antibodies can be obtained by using human hybridomas (Cote et al., 1983, Proc. Natl. Acad. Sci. (USA), 80: 2026-2030) or by transforming human B cells with EBV virus in vitro (Cole et al., 1985, in, "Monoclonal Antibodies and Cancer Therapy", Alan R. Liss, pp. 77-96). Techniques recently developed for the production of "chimeric antibodies" may be employed (Morrison et al., 1984, Proc. Natl. Acad. Sci.(USA)., 81: 6851-6855; Neuberger et al., 1984, Nature 312: 604-608; Takeda et al., 1985, Nature 314: 452-454). These latter techniques involve splicing the genes from a mouse antibody molecule of appropriate antigen specificity with genes from a human antibody molecule of appropriate biological activity.

A technique recently described can be used to generate a repertoire of monoclonal antibodies that define Oncostatin M (see Sastry et al., 1989, Proc. Natl. Acad. Sci. 86:5728-5732; and Huse et al., 1989, Science 246:1275-1281). Accordingly, a cDNA library of Fab fragments derived from splenic DNA of animals primed with Oncostatin M can be generated in bacterial host cells.

The monoclonal antibodies used in the invention can be modified by treatment with appropriate proteases, e.g., pepsin, papain, and the like, to generate Fab, F(ab')₂ or F_{V} fragments that immunospecifically bind to Oncostatin M.

Additionally, the whole antibody molecule or its Fab, F(ab')₂ or F_{V} fragment may be conjugated to any of a variety of compounds including, but not limited to, signal generating compounds such as a fluorescer, radiolabel, a chromophore, an enzyme, a chemoluminescent or bioluminescent molecule, etc. Alternatively, the whole antibody or its Fab, F(ab')₂ or F_{V} fragment may be conjugated to a growth factor which may enhance or inhibit the biological activity of Oncostatin M; or to toxins so that cells which express Oncostatin M precursors on their surface would be selectively killed. Methods which can be used for conjugating labels, proteins, toxins etc. to antibodies and antibody fragments are well known in the art. See, for example, U.S. Patent Nos. 4,220,450; 4,235,869; 3,935,074; and 3,996,345.

### USES OF MONOCLONAL ANTIBODIES TO ONCOSTATIN M

The antibodies may be advantageously used to detect native or denatured forms of natural or recombinant Oncostatin M or to detect the presence of Oncostatin M in serum samples where it may occur in a free form or associated with its binding protein. Alternatively, the antibodies may be used in vivo to inhibit the biological effects of Oncostatin M. Either polyclonal or monoclonal antibodies may be used for the detection of Oncostatin M in a sample, such as cells or physiological fluid, e.g., blood. Detection of Oncostatin M in a body fluid may also be used as an indication of the presence of a tumor cell. In this regard, anti-Oncostatin M antibodies may be useful in the diagnosis and/or prognosis of cancer and/or other cell growth-related disease. The antibodies may also be used in affinity chromatography for isolating and purifying Oncostatin M from natural or synthetic sources. The antibodies will find use also in controlling the amount of Oncostatin M associated with cells in culture or in vivo, whereby growth of the cells may be modified by the formation of a specific antibody: Oncostatin M complex resulting in competitive inhibition of Oncostatin M: Oncostatin M receptor binding. Thus, the antibodies may be useful as therapeutic agents in the treatment of cell growth disorders in which the growth stimulating activity of Oncostatin M is a factor.

### USE OF ANTI-ONCOSTATIN M TO INHIBIT GROWTH TO INHIBIT GROWTH OF KAPOSI'S SARCOMA

Of particular interest is the use of the anti-Oncostatin M antibodies in the invention for inhibiting the growth of cells stimulated by Oncostatin M. In particular the growth of a Kaposi's Sarcoma cell line assayed in vitro is shown infra to be potentiated by Oncostatin M. When neutralizing anti-Oncostatin M-antibodies are present in the assay, the potentiating effect of Oncostatin M is inhibited by the monoclonal antibodies. Thus, the neutralizing antibodies may be used therapeutically to treat Kaposi's Sarcoma. In this regard, a therapeutically effective dose of a neutralizing anti-Oncostatin M antibody, or anti-Oncostatin M fragment thereof, may be administered in vivo. Dosage schedules sufficient to achieve a serum concentration of the antibody, or a fragment thereof, that inhibits the effect of Oncostatin M as measured in vitro may be utilized.

### GENERATION OF ANTI-IDIOTYPES THAT MIMIC THE EFFECTS OF ONCOSTATIN M

The monoclonal antibodies can also be used to generate anti-idiotypic antibodies that mimic the biological effects of Oncostatin M. Anti-idiotypic antibodies or anti-idiotypes are antibodies directed against the antigen-combining region or variable region (called the idiotype) of another antibody molecule. In theory, based on Jerne's network model of idiotypic relationships (Jerne, N.K., 1974 Ann. Immunol. (Paris) 125:373:; Jerne, N.K. et al., 1982, EMBO 234), immunization with an antibody molecule expressing a paratope (antigen-combining site) for a given antigen should produce a group of anti-idiotypic antibodies, some of which share with the antigen a complementary structure to the paratope. Immunization with monoclonal antibodies that inhibit binding of Oncostatin M to its receptor should, in turn, produce anti-idiotypes that mimic Oncostatin M and bind to the Oncostatin M receptor. Thus, it is within the scope of the invention that these anti-idiotypes can be produced by the monoclonal antibodies directed against Oncostatin M which will mimic the effects of Oncostatin M in vivo and in vitro. Likewise, anti-idiotypic antibodies that bind to Oncostatin M are intended to be included in the definition of monoclonal antibodies that define Oncostatin M as used herein.

The following non-limiting Examples and Procedures are provided to further illustrate the invention.

### PRODUCTION OF MONOCLONAL ANTIBODIES TO ONCOSTATIN M

### A. IMMUNIZATION AND FUSION

The hybridomas were produced by immunization of 4 Balb/c mice with either 5 µg or 10 µg of recombinant Oncostatin M purified as described (Zarling, et al., 1986, Proc. Natl. Acad. Sci.(USA) 83:9739-97). Briefly, the Oncostatin M was resuspended in PBS, emulsified in an equal volume of Complete Freund's Adjuvant, and 25 µg of the emulsion was injected into one hind footpad intradermally. Two weeks later, the same immunization protocol in the same hind footpad was followed, with concentrations of Oncostatin-M identical to that for the first immunization. Two weeks after the second immunization, the mice were injected in the same hind footpad with a third preparation of Oncostatin-M, this time emulsified in Incomplete Freund's Adjuvant. Three days after the final immunization, the popliteal lymph node was removed, and the lymph node cells were fused with murine myeloma 653/AG8 cells at a 1:1 lymph node cell to myeloma cell ratio, using 40% polyethylene glycol as a fusion agent. Fusion products, at a concentration of 5 X 10⁴ cells/well, were plated into 96-well plates containing hypoxanthine-amitnopterin-thymidine as selection agent, in Iscoves Modification of Dulbecco's Modified Eagle's Medium (IDMEM) supplemented with 10% fetal bovine serum, sodium pyruvate, and L-glutamine. After one week, original medium was replaced with fresh medium containing selection agent. Hybrids were screened by a solid-phase Micro Enzyme-linked Assay ("Micro EIA"), as described below, when the hybrids were visible macroscopically.

### B. MICRO EIA

The following Micro EIA was used to characterize the specificity of the antibodies to Oncostatin M produced by the hybridomas. Hybridoma supernatants were screened for activity using a solid-phase EIA adapted for use in Microtest plates (Robbins Scientific, San Francisco). Concentrations of purified Oncostatin-M ranging from 2 to 4 µg/ml were plated in 5 µl volumes in each well, and allowed to air-dry overnight. After a one hour block with 5% nonfat dried milk in PBS and 0.1% sodium azide, the hybrid supernatants were added in 1 µl volumes, and incubated at room temperature for 1 hour. The plates were washed six times by immersion in PBS. Peroxidase-conjugated F(ab')₂ goat anti-mouse IgG (Pel-Freez, Rogers AK) in PBS+2% BSA at a concentration of 1:1500 was added in 5 µl volumes to each well. After a 1 hour incubation, the plates were washed six times as previously described; 10 µl of substrate, 2,2'-azino-bis-(3-ethylbenzthiazoline-6-sulfonic acid) ("ABTS"), (Sigma Chemical Co., St. Louis Mo.) was added, and plates were read at OD = 660 nm after 20 minutes. Positive wells were determined on the basis of signal above background relative to the signal of a previously generated rabbit antiserum against Oncostatin-M. Positive hybrids were cloned in soft agar.

### C. IMMUNOPRECIPITATION

CHO cells transfected with the Oncostatin-M-encoding cDNA were incubated for 4-8 hr with 200 µCi each of ³⁵S- methionine and ³⁵S-cysteine in a volume of 4 ml in a 60 x 15 cm petri dish. Supernatants were collected and filtered; 10mM phenylmethylsulfonylfluoride ("PMSF") and 100mM tosyl-lysine-chloromethylketone ("TLCK") were added to inhibit proteases. For immunoprecipitations, 100-200 µl of labelled supernatant were incubated overnight at 4°C with rotating with 200 µl of spent supernatant from the monoclonal antibody-secreting hybrids. Antigen-antibody complexes were isolated by incubation with a monoclonal rat anti-mouse κ light chain monoclonal antibody covalently coupled to Reactigel beads (Pierce Chemicals). After washing, the antigen-antibody complexes were eluted by boiling in sample buffer containing 1% SDS and 5% 2-mercaptoethanol. The eluted material was electrophoresed in 15% SDS-PAGE. Gels were fluorographed with Amplify™ (Amersham Corp., Arlington Heights, IL.), dried, and autoradiographed with Kodak X-AR5 X-ray film.

### D. GROWTH INHIBITION ASSAY

The antibodies were tested to determine whether any could neutralize the inhibitory effects of Oncostatin-M in a Growth Inhibitory Assay ("GIA"). The GIA employs A375 melanoma cells (4x10³ cells/50 µl) as indicator cells. A375 cells are subcultured for four hours on flat-bottomed 96-well tissue culture plates (Costar 3595, Cambridge, MA) in growth medium comprising Dulbecco's modified Eagle's medium ("DMEM"), supplemented with 10% heat inactivated fetal bovine serum, and penicillin/streptomycin. Oncostatin M is diluted in growth medium and assayed for growth inhibition in triplicate by adding 50 µl of the diluted sample per well. The cells then are incubated for 72 hours at 37°C. At the end of this incubation period, each well is treated for 24 hours with 100 µl of growth medium containing [¹²⁵I]iodo-2'-deoxyuridine (0.05 µCi/well (Amersham Corp., Arlington Heights, IL). The monolayers are washed with phosphate-buffered saline ("PBS"), fixed in 95% methanol and air-dried. The [¹²⁵I]-iododeoxyuridine incorporated by the cells is solubilized with 200 µl of 1 N sodium hydroxide and the amount of cell growth measured by the amount of [¹²⁵I]iododeoxyuridine incorporated into the DNA of actively growing cells. One unit of activity is defined as the amount of the Oncostatin M required to give 50% inhibition of growth of A375 cells relative to untreated cells.

### E. ONCOSTATIN M RADIORECEPTOR ASSAY

Antibodies were tested in a radioreceptor assay for the ability to inhibit the binding of ¹²⁵I-labeled Oncostatin-M to cell surface receptors for Oncostatin M on the human carcinoma cell line H2981. H2981 cells are plated in 48-well plates at a density of 2x10⁵ cells/well and maintained at 37°C for 16-24 hours before initiation of the assay. Cell monolayers are then washed once with Binding Buffer (Linsley et al., 1986, Biochemistry 25:2978-2986). To measure total binding, ¹²⁵I-Oncostatin M was added at concentrations ranging from 0.5-100 ng/ml. To measure non-specific binding, unlabeled Oncostatin M is added simultaneously with the ¹²⁵I-Oncostatin M to replicate plates at a concentration 20 to 100-fold higher than the concentration of ¹²⁵I-Oncostatin M. Binding is allowed to proceed for 2-5 hours at 23°C, then the monolayers are washed four times with Binding Buffer. Cell-bound radioactivity is solubilized with 1 N NaOH and counted in a gamma counter. Specific binding is calculated by subtracting the non-specific binding from total binding. The dissociation constant (K_{d}) and binding capacity was determined by Scatchard analysis (Scatchard, Ann. N.Y. Acad. Sci. (1949) 51:660).

### F. ONCOSTATIN M MUTANTS

Anti-Oncostatin M monoclonal antibodies were tested against a series of recombinant Oncostatin M mutants, having insertions, deletions or substitutions which were generated at the DNA level. The antibodies were tested as spent supernatants in an EIA on media from COS cells which were transfected transiently with the various parental and mutant constructs.

### G. DETECTION OF ONCOSTATIN M IN SERUM WITH MONOCLONAL ANTIBODIES

The presence of Oncostatin-M in serum was detected as follows: Human serum samples were first applied to an S-300 sizing column to separate serum proteins by molecular weight. Fractions collected from the S-300 column were examined for GIA activity, both before and after acidification of the serum samples. Before acidification, most serum samples exhibited no GIA activity in the void volume fractions. However, serum fractions which were acidified, then reneutralized before adding to the GIA, exhibited activity in fractions of molecular weights greater than 200 kilodaltons, suggesting that the Oncostatin-M in serum is generally associated with a binding factor of greater than 200kd, and is inactive in this state. The various serum fractions were examined by Western blotting. Reaction of the Oncostatin-M-specific monoclonal antibodies with proteins corresponding to the molecular weight of Oncostatin-M, but found in the high molecular weight fractions, confirmed the presence of Oncostatin-M in these fractions. The Oncostatin-M found in serum has been purified, and N-terminal amino acid sequencing indicates that it is identical to native and recombinant Oncostatin-M previously obtained.

### RESULTS

### H. SELECTION OF MONOCLONAL ANTIBODIES WITH SPECIFICITY FOR ONCOSTATIN M

Two series of fusions were performed, consisting of four fusions in each series, with one lymph node from each mouse used per fusion. From the first series, four hybrids were examined more extensively. From the second, more successful series of fusions (in terms of numbers of positive hybrids identified in the MicroEIA), at least 20 other anti-Oncostatin-M monoclonal antibodies were identified. From this number, the following hybrids were selected for further study based on relative signal in the EIA and ability to immunoprecipitate either native or denatured metabolically labelled Oncostatin-M: 11R2F8, 12R13B5, 12R13D7, and 12R19E3. Based on the immunoprecipitation data described below, the hybrids were grouped into one of three categories based on their reactivities with either native Oncostatin M, denatured Oncostatin M, or both (Table I). Five of these monoclonal antibodies were then tested to determine whether any of them could neutralize the inhibitory effects of Oncostatin-M in the Growth Inhibitory Assay. These results are described below.

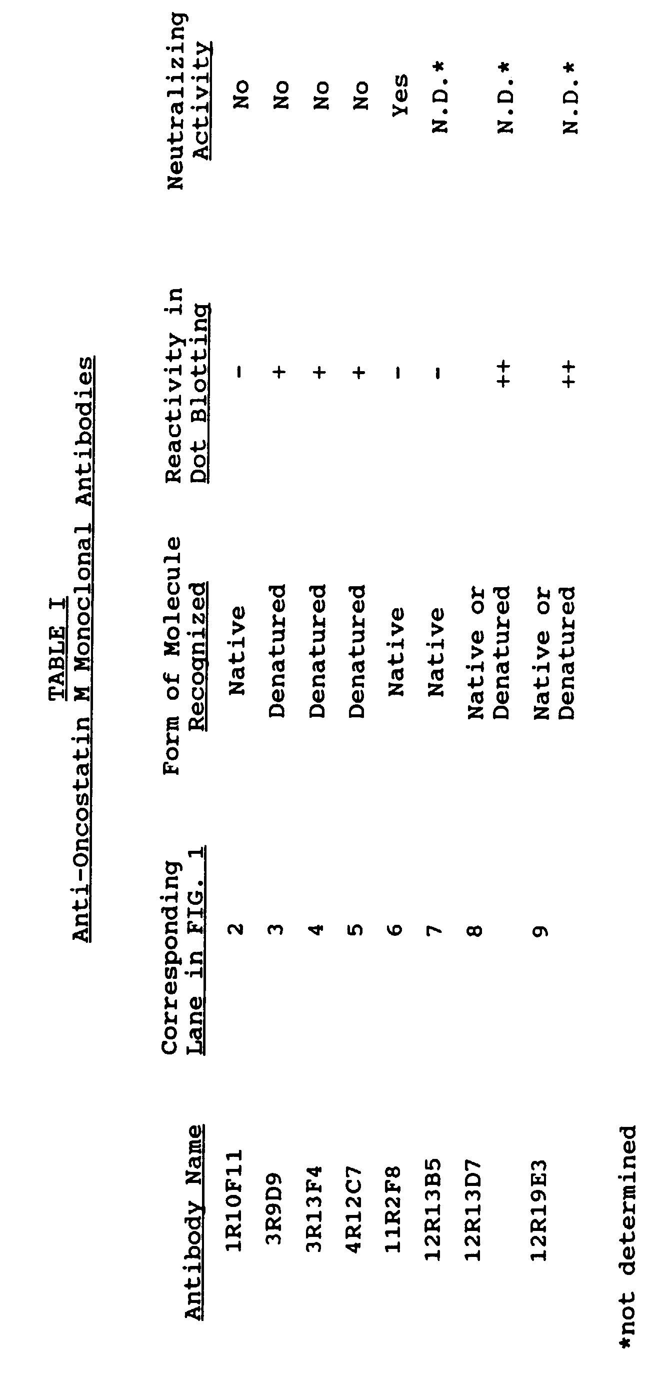

### I. MONOCLONAL ANTIBODIES IMMUNOPRECIPITATE WITH ONCOSTATIN M

Results of immunoprecipitation data identified three hybridomas reacting only with ³⁵S-methionine-labelled Oncostatin-M in the form in which it was secreted from the CHO transfectant (group 1) (lanes 2,6,7; Figure 1; see Table I for identification of antibodies). Three additional monoclonal antibodies reacted only with ³⁵S-methionine-labelled Oncostatin-M which was first reduced and denatured by treating with SDS and reducing agents, then boiled at 100°C, but not with native Oncostatin-M (group 2, lanes 3,4,5). Two additional monclonal antibodies reacted with biosynthetically labelled Oncostatin-M whether it was native or denatured (group 3, lanes 8,9).

### J. MONOCLONAL ANTIBODIES THAT NEUTRALIZE ONCOSTATIN M IN THE GROWTH INHIBITION ASSAY

Purified antibody from the hybrid 11R2F8 neutralized the effects of Oncostatin-M in a concentration-dependent fashion (Figure 2A). Antibody 1R10F11, which is directed against native Oncostatin-M, showed no blocking of growth inhibitory effects of Oncostatin-M (Figure 2B). None of the antibodies detecting a denatured determinant was capable of neutralizing Oncostatin-M as measured by GIA. The neutralizing antibody 11R2F8 was further tested for its ability to inhibit Oncostatin-M receptor binding, as described below.

### K. MONOCLONAL ANTIBODIES THAT INHIBIT BINDING OF ONCOSTATIN M IN THE RADIORECEPTOR ASSAY

The 11R2F8 antibody was capable of inhibiting Oncostatin-M receptor binding in a concentration dependent fashion, in contrast to either the 1R10F11 antibody, which is reactive with a native but non-neutralizing site, or a third antibody, 4R12C7, directed against an epitope on denatured Oncostatin-M (Figure 3).

### L. MONOCLONAL ANTIBODIES TESTED AGAINST RECOMBINANT ONCOSTATIN M MUTANTS

Oncostatin M mutants are described in WO-A- 9109057. Five different antibodies were tested on each Oncostatin M mutant. The binding of an antibody detecting a denatured Oncostatin-M determinant, 4R12C7, is compared with that of the neutralizing monoclonal antibody 11R2F8 (Figure 4). As increasing numbers of amino acids are deleted from the C terminal portion of the recombinant Oncostatin-M molecule, there is a decrease in reactivity of the two antibodies directed against native epitopes, 1R10F11, and 11R2F8, on these constructs. When amino acid residue number 183 is deleted, activities of both antibodies are lost, implying that the tertiary structure recognized by these antibodies somehow involves amino acids located in the C-terminal portion of the molecule. In all but one mutant construct examined, the two antibodies directed against native Oncostatin-M have identical reactivity. The lone exception, construct Δ44-47, is bound by 1R10F11, but not by 11R2F8, the neutralizing antibody, suggesting that the conformational change resulting from this mutation destroys the neutralizing site but not the site recognized by 1R10F11. The EIA data were confirmed by immunoprecipitation analysis of biosynthetically labelled media from the COS transfectants (Figure 5).

The C-terminal deletion mutant termed Δ187 was informative in the mapping of the binding site of 12R13D7 (Figure 1, lane 8), an antibody which reacts with both native and denatured Oncostatin-M. This antibody reacts with all of the other mutant constructs so far expressed except Δ187. Since 12R13D7 reacts with Δ188 and Δ186, which have deletions of amino acids preceeding and following the Δ187 mutation, it was suspected that a mutation elsewhere in Δ187 was responsible for the loss of reactivity of this antibody. When Δ187 was sequenced, an additional mutation was found at amino acid 108, changing a leucine to a serine, thus localizing the binding site of the 12R13D7 antibody to this region.

### M. ANTI-ONCOSTATIN M INHIBITS GROWTH POTENTIATING EFFECTS OF ONCOSTATIN M ON KAPOSI'S SARCOMA DERIVED AIDS-KS CELLS

The growth potentiating activity of Oncostatin M on Kaposi's Sarcoma is illustrated in Figure 6. Purified Oncostatin M increased cellular growth of the Kaposi's Sarcoma cell line as well as of N1072, smooth muscle cells derived from the pleural effusion of a patient with malignant mediastinal lymphoma, cultured in vitro. Neutralizing monoclonal antibody 11R2F8 of the invention, added to the Kaposi Sarcoma culture system, inhibited the growth potentiating activity of the exogenous Oncostatin M by 95% (Figure 7).

Conditioned medium from an HTLV-II infected cell line (#38) also potentiates the growth of Kaposi Sarcoma cells (see Nakamura et al., 1988, Science 242:426-430). Monoclonal antibody 11R2F8 also inhibits the growth potentiating activity of such conditioned medium on Kaposi's Sarcoma cells. As shown in Figure 7, 60% inhibition of the Oncostatin M activity is obtained.

The non-neutralizing monoclonal antibody 1R10F11 had no effect on the growth stimulating activity of Oncostatin M in either assay (Figure 7).

### N. DEPOSIT OF MICROORGANISMS

The following cell lines, representative of the use in present invention, have been deposited with the American Type Culture Collection, Rockville, Maryland, and have been assigned the listed accession numbers:

| Cell Line | Accession Number | Date of Deposit |
|---|---|---|
| 11R2F8.9 | ATCC HB 10398 | March 27, 1990 |
| 12R13D7.2 | ATCC HB 10396 | March 27, 1990 |
| 1R10F11.34.16 | ATCC HB 10397 | March 27, 1990 |

The present invention is not to be limited in scope by the cell lines deposited since the deposited embodiments are intended as single illustrations of individual aspects of the invention, and any cell line or antibody which is functionally equivalent is within the scope of use of this invention. Indeed, various modifications of the invention in addition to those shown and described will become apparent to those skilled in the art from the foregoing description and accompanying drawings. Such modifications are intended to fall within the scope of the appended claims.

## Claims

1. The use of an antibody, or fragment thereof, the antigen-combining site of which is reactive with Oncostatin M, for preparing a pharmaceutical composition which inhibits the growth of Kaposi's Sarcoma cells.

2. The use of Claim 1, wherein the antibody inhibits Oncostatin M receptor binding.

3. The use of Claims 1 or 2 wherein the antibody is a monoclonal antibody, or fragment thereof.

4. The use of Claim 3 wherein the monoclonal antibody is an Fab, F(ab')₂ or Fv fragment.

5. The use of any one of Claims 1 to 4 wherein the antibody is conjugated to a label, a toxin, or a growth factor.

6. The use of an antibody, or fragment thereof, reactive with Oncostatin M, the antigen-combining site of said antibody or fragment thereof being capable of competitively inhibiting the immunospecific binding of monoclonal antibody 11R2F8 (ATCC 10398), for preparing a pharmaceutical composition which inhibits the growth of Kaposi's Sarcoma cells.

7. The use of claim 6, wherein the antibody, or fragment thereof, is a chimeric antibody, or fragment thereof.

8. The use of claim 1 wherein the antibody is monoclonal antibody 11R2F8 (ATCC 10398).

9. The use of claims 6 to 8 wherein an Fab, F(ab')₂ or Fv fragment of the monoclonal antibody is applied.

10. The use of claim 9, wherein the antibody fragment is conjugated to a label, a toxin, or a growth factor.

11. The use of one of the preceding claims, wherein a chimeric antibody, or fragment thereof, is applied at least an antigen-combining site of which is derivable from monoclonal antibody 11R2F8 (ATCC 10398).

## Patentansprüche

1. Verwendung eines Antikörpers oder eines Fragmentes davon, dessen Antigen-Bindungsstelle reaktiv gegenüber Oncostatin M ist, zur Herstellung eines pharmazeutischen Mittels, welches das Wachstum von Kaposi-Sarkomzellen inhibiert.

2. Verwendung nach Anspruch 1, wobei der Antikörper die Oncostatin M-Rezeptorbindung inhibiert.

3. Verwendung nach Anspruch 1 oder 2, wobei der Antikörper ein monoklonaler Antikörper oder ein Fragment davon ist.

4. Verwendung nach Anspruch 3, wobei der monoklonale Antikörper ein Fab-, F(ab')₂- oder Fv-Fragment ist.

5. Verwendung nach einem der Ansprüche 1 bis 4, wobei der Antikörper mit einem Marker, einem Toxin oder einem Wachstumsfaktor konjugiert ist.

6. Verwendung eines gegenüber Oncostatin M reaktiven Antikörpers oder eines Fragmentes davon, wobei die Antigen-Bindungsstelle des Antikörpers oder des Fragmentes davon zur kompetitiven Inhibierung der immunospezifischen Bindung des monoklonalen Antikörpers 11R2F8 (ATCC 10398) befähigt ist, zur Herstellung eines pharmazeutischen Mittels zur Inhibierung des Wachstums von Kaposi-Sarkomzellen.

7. Verwendung nach Anspruch 6, wobei der Antikörper oder das Fragment davon ein chimärer Antikörper oder ein Fragment davon ist.

8. Verwendung nach Anspruch 1, wobei es sich bei dem Antikörper um den monoklonalen Antikörper 11R2F8 (ATCC 10398) handelt.

9. Verwendung nach den Ansprüchen 6 bis 8, wobei ein Fab-, F(ab')₂- oder Fv-Fragment des monoklonalen Antikörpers zur Anwendung kommt.

10. Verwendung nach Anspruch 9, wobei das Antikörperfragment mit einem Marker, einem Toxin oder einem Wachstumsfaktor konjugiert ist.

11. Verwendung nach einem der vorhergehenden Ansprüche, wobei ein chimärer Antikörper oder ein Fragment davon zur Anwendung kommt, wobei wenigstens eine Antigen-Bindungsstelle davon von dem monoklonalen Antikörper 11R2F8 (ATCC 10398) ableitbar ist.

## Revendications

1. Utilisation d'un anticorps, ou fragment de celui-ci, dont le site de combinaison d'antigène est réactif avec l'Oncostatine M, pour préparer une composition pharmaceutique qui inhibe la croissance des cellules du sarcome de Kaposi.

2. Utilisation de la revendication 1 où l'anticorps inhibe la liaison du récepteur de l'Oncostatine M.

3. Utilisation de la revendication 1 ou 2 où l'anticorps est un anticorps monoclonal ou un fragment de celui-ci.

4. Utilisation de la revendication 3 où l'anticorps monoclonal est un fragment Fab, F(ab')₂ ou Fv.

5. Utilisation selon l'une quelconque des revendications 1 à 4 où l'anticorps est conjugué à un marqueur, une toxine ou un facteur de croissance.

6. Utilisation d'un anticorps ou fragment de celui-ci, réactif avec l'Oncostatine M, le site de combinaison de l'antigène dudit anticorps ou un fragment de celui-ci étant capable d'inhiber compétitivement la liaison immunospécifique de l'anticorps monoclonal 11R2F8 (ATCC 10398) pour la préparation d'une composition pharmaceutique qui inhibe la croissance des cellules du sarcome de Kaposi.

7. Utilisation de la revendication 6 où l'anticorps ou fragment de celui-ci est un anticorps chimérique ou fragment de celui-ci.

8. Utilisation de la revendication 1 où l'anticorps est l'anticorps monoclonal 11R2F8 (ATCC 10398).

9. Utilisation des revendications 6 à 8 où un fragment Fab, F(ab')₂ ou Fv de l'anticorps monoclonal est appliqué.

10. Utilisation de la revendication 9 où le fragment d'anticorps est conjugué à un marqueur, une toxine ou un facteur de croissance.

11. Utilisation selon l'une des revendications précédentes où un anticorps chimérique, ou fragment de celui-ci, est appliqué à un site de combinaison d'antigène qui est dérivable de l'anticorps monoclonal 11R2F8 (ATCC 10398).
